(19) Europäisches Patentamt
European Patent Office
Office européen des brevets

(11) Veröffentlichungsnummer: **0 090 946**
**B1**

(12) **EUROPÄISCHE PATENTSCHRIFT**

(45) Veröffentlichungstag der Patentschrift: **02.10.85**

(51) Int. Cl.⁴: **C 01 F 7/00**

(21) Anmeldenummer: **83101914.6**

(22) Anmeldetag: **26.02.83**

(54) **Verfahren zur Herstellung von Dihydroxialuminiumnatriumcarbonat.**

(30) Priorität: **06.04.82 DE 3212799**

(43) Veröffentlichungstag der Anmeldung: **12.10.83 Patentblatt 83/41**

(45) Bekanntmachung des Hinweises auf die Patenterteilung: **02.10.85 Patentblatt 85/40**

(84) Benannte Vertragsstaaten: **AT BE CH DE FR GB IT LI LU NL SE**

(56) Entgegenhaltungen:
**DE-B-2 708 861**
**GB-A-1 174 889**
**US-A-2 783 179**
**Chemical Abstracts, Band 76, 1972, Columbus, Ohio, USA**
**Chemical Abstracts, Band 78, 1973, Columbus, Ohio, USA**
**Chemical Abstracts, Band 78, 1973, Columbus, Ohio, USA**
**Chemical Abstracts, Band 88, 1978, Columbus, Ohio, USA**
**Chemical Abstracts, Band 75, 1971, Columbus, Ohio, USA**

**Die Akte enthält technische Angaben, die nach dem Eingang der Anmeldung eingereicht wurden und die nicht in dieser Patentschrift enthalten sind.**

(73) Patentinhaber: **Giulini Chemie GmbH, Giulinistrasse 2, D-6700 Ludwigshafen/Rhein (DE)**

(72) Erfinder: **Urmann, Ernst, Dr. Dipl.-Chem., Limesstrasse 3, D-6700 Ludwigshafen/Rhein (DE)**
Erfinder: **Grund, Hartmut, Dr. Dipl.-Chem., Franz Schubert-Strasse 13, D-6701 Waldsee/Pf. (DE)**
Erfinder: **Drautzburg, Günter, Dr. Dipl.-Chem., Lindenbergerstrasse 23, D-6700 Ludwigshafen/Rhein (DE)**
Erfinder: **Schanz, Klaus, Dr. Dipl.-Chem., In der Zeil 5, D-6701 Dannstadt-Schauernheim (DE)**

(74) Vertreter: **Benatzky, Erika, Dr., Giulinistrasse 2 Postfach 150480, D-6700 Ludwigshafen/Rh. (DE)**

**EP 0 090 946 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der vorliegenden Erfindung ist ein verbessertes Verfahren zur Herstellung von Dihydroxialuminiumnatriumcarbonat aus Natriumaluminatlösung.

Dihydroxialuminiumnatriumcarbonat, als Mineral und hier auch Dawsonit genannt, ist — wie seine Verwendung als Antazidum — schon seit langem bekannt. Zu seiner Herstellung sind auch bereits eine Anzahl von Verfahren vorgeschlagen worden, von denen einige in der Praxis ausgeübt werden.

Gemäß der DE-A-2 708 861 wird Dawsonit durch Reaktion von Aluminiumhydroxid mit einer wäßrigen Natriumbicarbonatlösung bei einer Temperatur zwischen 160 und 240° C und unter einem Druck zwischen 5 und 50 atm. (5 und 50 bar) hergestellt.

Die Umsetzung von Natriumbicarbonat mit Aluminiumalkoxid oder Natriumaluminat in einem Wasser-Alkohol-Gemisch ist Gegenstand der US-A-2 783 179. Während bei der Reaktion mit Aluminiumalkoxid das im wesentlichen amorphe Dihydroxialuminiumnatriumcarbonat entsteht, wird bei der Umsetzung mit Natriumaluminat die isomere Verbindung Hydroxinatriumoxialuminiumhydrogencarbonat

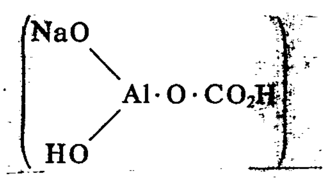

gebildet, die in ihrem chemischen Verhalten von dem des Isomeren im Verlauf der Titrationskurve abweicht.

Ein Nachteil des vorstehenden Herstellungsverfahrens wird bei beiden Varianten darin gesehen, daß sie in reinem Wasser undurchführbar sind. Während bei der ersten Verfahrensvariante die kostenintensiveren Aluminiumalkoxide als Ausgangsprodukte eingesetzt werden, müssen bei der zweiten erhebliche Alkoholmengen in das Reaktionsmedium eingeschleust werden. Verzichtet man auf die Alkoholzugabe, tritt rasch Hydrolyse unter Bildung von Aluminiumhydroxid und anderen Produkten ein.

Es ist auch bereits vorgeschlagen worden, Dawsonit durch Einleiten von $CO_2$ in Natriumaluminatlösung zu bilden. Dabei wurde festgestellt, daß die Zersetzung wäßriger Natriumaluminatlösungen durch Kohlendioxideinleitung sowohl vom Molverhältnis $Na_2O/Al_2O_3$ als auch von der Temperatur stark beeinflußt wird. Bei niedrigem Molverhältnis und niedriger Temperatur entsteht demnach Aluminiumhydroxidgel und Bayerit. Temperatursteigerung führt bei gleichem Molverhältnis $Na_2O/Al_2O_3$ zu Böhmit und Dawsonit. Wird nur das Molverhältnis erhöht, erhält man Böhmit. Höhere Dawsonitausbeuten sollen jedoch bei einem Molverhältnis $Na_2O/Al_2O_3$ größer als 4 und Temperaturen zwischen 50 und 90° C erzielt werden (Chem. Abstr. Vol. 75, 153737 u (1971)).

Wie nunmehr festgestellt wurde, wird die Dawsonitausbeute bei dem vorstehenden Verfahren nicht nur von Molverhältnis und Temperatur stark beeinflußt, sondern auch von der Geschwindigkeit der Kohlensäurezufuhr. Außerdem sind nur Entfernung der überschüssigen Alkalien umfangreiche Waschvorgänge erforderlich, so daß reine und hochwirksame Antazida auf Dawsonitbasis nur unter erheblichem Zeit- und Energieaufwand erzielbar sind.

Es stellte sich somit die Aufgabe, die Nachteile der vorstehenden Verfahren zu beseitigen und ein Verfahren zur Dawsonitgewinnung zu finden, das wesentlich einfacher, billiger und im Hinblick auf das Verfahrensprodukt sicherer durchführbar ist.

Die Lösung der gestellten Aufgabe besteht darin, daß man zunächst in Wasser, in welchem gegebenenfalls Aluminiumhydroxidgel suspendiert ist, bei Raumtemperatur (20—35° C) Kohlendioxid bis zur Sättigung einleitet, dann bei Einhaltung der Reaktionstemperatur unter weiterer Kohlendioxideinleitung kontinuierlich wäßrige Natriumaluminatlösung mit einem Molverhältnis $Na_2O/Al_2O_3$ von 1 : 1 bis 1,25 : 1 zugibt, und zwar derart, daß der pH-Wert während der Fällung in der Suspension stets unterhalb 9,8 bleibt und vorzugsweise zwischen 9,8 und 8,8 liegt und anschließend das Fällungsprodukt nach pH-Wert-Einstellung mit Kohlendioxid auf 7 bis 7,5 aus der Suspension abtrennt und bei erhöhter Temperatur trocknet.

Die Reaktionstemperatur liegt bei dem neuen Verfahren bei Raumtemperatur, also zwischen 20 und 35° C. Da auch die Umsetzung unter den angegebenen Verfahrensbedingungen bereits optimale oder nahezu optimale Ergebnisse bringt, erübrigt sich die Anwendung von Drükken, so daß der anlagentechnische Aufwand minimal ist.

Erfindungsgemäß liegt das Molverhältnis $Na_2O/Al_2O_3$ der Natriumaluminatlösung zwischen 1 : 1 und 1,25 : 1. Obwohl Natriumaluminatlösungen mit einem Molverhältnis von 1 : 1 herstellbar sind, arbeitet man zweckmäßigerweise mit einem höheren Molverhältnis. Unter den vorgegebenen Verfahrensbedingungen, insbesondere Konzentration, sind nämlich derartige Lösungen nicht ausreichend stabil. Das für eine stöchiometrische Zusammensetzung fehlende Aluminium kann erfindungsgemäß in Form von Aluminiumhydroxidgel zugeführt werden.

Natriumaluminatlösungen, die mit Vorteil eingesetzt werden, enthalten 140 bis 180 g $Al_2O_3$/l und 96 bis 123 g $Na_2O$/l. Das Molverhältnis sollte dabei auf 1,05 bis 1,15 eingestellt werden.

Ein wesentliches Verfahrensmerkmal besteht darin, daß die wäßrige Suspension während der Dawsonitfällung mit Kohlendioxid stets gesättigt ist. Abweichungen von diesem Sättigungsgrad führen zur Ausfällung von Aluminiumhydroxid in einer antazid wenig wirksamen Form. Wird unter

den vorgegebenen Bedingungen gearbeitet, wird ein 100%iger Umsatz erzielt. Man erhält ein Produkt, das gut suspendiert, keine Verunreinigungen enthält und nicht amorph ist.

An dieser Stelle ist noch hervorzuheben, daß das überschüssige Kohlendioxid bei dem neuen Verfahren aus wirtschaftlichen und sicherheitstechnischen Gründen rezirkuliert wird, also in einem geschlossenen System mit Absaug- und Fördervorrichtungen gearbeitet wird.

Ein besonderer Vorteil des neuen Herstellungsverfahrens liegt nun darin, daß die Suspensionen in besonders einfacher Weise in den festen Zustand überführt werden können. So kann die gebildete Suspension, unmittelbar nach Beendigung der Kohlensäureeinleitung ohne Filtration und ohne Waschen des Feststoffes in einen Sprühtrockner gepumpt und bei Eintrittstemperaturen von 280 bis 290° C und Austrittstemperaturen von 90 bis 95° C getrocknet werden. Selbstverständlich ist es auch möglich, den Feststoff durch andere Abtrennungsverfahren, z. B. Filter, Zentrifugen, Zyklone und Sedimentationsgefäße zu isolieren und nach anderen bekannten Verfahren zu trocknen.

Anhand der nachstehenden schematischen Zeichnung und der Beispiele soll das erfindungsgemäße Verfahren noch näher erläutert werden.

Der zur Dawsonitbildung vorgesehene Reaktor ist in der Zeichnung mit 1 bezeichnet. Er ist mit dem Rührer 2 und der Begasungseinrichtung 3 ausgestattet. Die Wasserzugabe erfolgt über die Rohrleitung 4 aus dem Behälter 5 und die Kohlendioxidzugabe über die Leitung 6, die an den Druckbehälter 9 zur $CO_2$-Entnahme angeschlossen ist. Das nicht verbrauchte $CO_2$ wird über Gebläse und Leitung 17 rezirkuliert. Die Natriumaluminatlauge wird dem Behälter 10 entnommen und über 11 in den Reaktor geführt. Der Reaktor ist über die Leitung 12 mit dem Sprühtrockner 13 verbunden, in welchen die Suspension gepumpt (14) wird. Mit 15 ist ein Staubfilter angedeutet und mit 16 die Gaszuführung in den Sprühtrockner.

Beispiel 1

20 l Wasser werden in einem Rührbehälter, der mit einer Begasungseinrichtung ausgestattet ist, bei Raumtemperatur (20° C) mit $CO_2$ gesättigt. Anschließend werden in das kohlensäurehaltige Wasser 12 l Natriumaluminatlösung eindosiert, etwa 20 cm³ in der Minute. Während des gesamten Fällungsvorganges, für den 10 Stunden benötigt werden, wird in die Suspension $CO_2$ eingeleitet. Der pH-Wert der Suspension wird zwischen 8,8 und 9,5 gehalten, er darf zu keinem Zeitpunkt größer als 9,8 sein.

Der $Al_2O_3$-Gehalt beträgt im Beispiel 155 g/l und der $Na_2O$-Gehalt 108 g/l (Molverh. $Na_2O/Al_2O_3 = 1,14$). Das ausfallende Natriumaluminiumdihydroxicarbonat ist mikrokristallin. Bei Beendigung der Aluminatzugabe wird der pH-Wert durch Einleiten von $CO_2$ auf 7,2 abgesenkt. Die Suspension wird abfiltriert und das Gut im Pastentrockner bei 60° C getrocknet.

Beispiel 2

In einem Rührbehälter, der zur Einführung des Kohlendioxids am Boden eine ringförmige Leitung mit einem geeigneten Düsensystem enthält, werden 14 m³ Wasser, in dem 51,3 kg Aluminiumhydroxidgel suspendiert sind, vorgelegt und bei RT. mit $CO_2$ gesättigt. Unter weiterem Einleiten von $CO_2$ werden in die Wasservorlage 5,5 m³ Natriumaluminatlösung mit einem Gehalt von 120 g $Na_2O$/l und 152 g $Al_2O_3$/l zudosiert, und zwar derart, daß der pH-Wert stets unter 9,5 bleibt. Das Molverhältnis $Na_2O/Al_2O_3$ in der Natriumaluminatlösung beträgt 1,25 und die Reaktionszeit 5,5 Stunden. Die gebildete Suspension weist eine Feststoffkonzentration von 153 g/l auf und wird nach pH-Wertabsenkung auf 7,5 sofort in einen Sprühtrockner gepumpt. Die Eintrittstemperatur der Luft beträgt in dem Sprühtrockner der Fa. NIRO-ATOMIZER 280 bis 290° C, die Austrittstemperatur 90 bis 95° C.

Das sprühgetrocknete Produkt hat folgende Zusammensetzung:

| | |
|---|---|
| $Al_2O_3$ | 29 Gew.-% |
| $Na_2O$ | 19 Gew.-% |
| $CO_2$ | 28 Gew.-% |
| Feuchte | 14 Gew.-% |

Mindestens 90% der Teilchen zeigen eine Größe kleiner 30 µm. Die Säurebindungskapazität des im Beispiel hergestellten Dihydroxidaluminiumnatriumcarbonats wird gemäß SJOEGREN-Test bestimmt, bei dem die Säurebindungsfähigkeit pro g suspendiertem Antazidum in Abhängigkeit von der Zeit unter pH-Wert-Konstanthaltung (pH = 3) mit $0,1_n$ HCl/g gemessen wird. Der HCl-Verbrauch liegt

nach 5 Min. bei 189 ml ≙ 81,5% d. Th.
nach 10 Min. bei 219 ml ≙ 94,4% d. Th.
nach 30 Min. bei 229 ml ≙ 98,7% d. Th.

Die Säurebindungskapazität des Dihydroxinatriumaluminiumnatriumcarbonats, bestimmt nach der Methode der USP XIX, beträgt 230 ml $0,1_n$ HCl/g, was einem Wert von 99,13% des theoretischen Säurebindungsvermögens entspricht.

**Patentansprüche**

1. Verfahren zur Herstellung von Dihydroxialuminiumnatriumcarbonat durch Umsetzung von wäßriger Natriumaluminatlösung mit Kohlendioxid und anschließender Abtrennung und Trocknung des Reaktionsproduktes, dadurch gekennzeichnet, daß man zunächst bei einer Reaktionstemperatur zwischen 20 und 35°C in Wasser Kohlendioxid bis zur Sättigung einleitet, dann

bei Einhaltung der Reaktionstemperatur unter weiterer Kohlendioxideinleitung kontinuierlich wäßrige Natriumaluminatlösung mit einem Molverhältnis $Na_2O/Al_2O_3$ von 1 : 1 bis 1,25 : 1 zugibt, und zwar derart, daß der pH-Wert während der Fällung in der Suspension stets unterhalb 9,8 bleibt, und vorzugsweise zwischen 9,8 und 8,8 liegt und anschließend das Fällungsprodukt nach pH-Wert-Einstellung mit Kohlendioxid auf 7 bis 7,5 aus der Suspension abtrennt und bei erhöhter Temperatur trocknet.

2. Verfahren nach Anspruch 1, dadurch gekennzeichnet, daß eine Natriumaluminatlösung mit einem Molverhältnis $Na_2O/Al_2O_3$ von 1,05 : 1 bis 1,15 : 1 eingesetzt wird.

3. Verfahren nach den Ansprüchen 1 und 2, dadurch gekennzeichnet, daß eine Natriumaluminatlösung mit

96 bis 123 g $Na_2O$/l und
140 bis 180 g $Al_2O_3$/l eingesetzt wird.

4. Verfahren nach den Ansprüchen 1 bis 3, dadurch gekennzeichnet, daß die Umsetzung bei erhöhtem Druck durchgeführt wird.

5. Verfahren nach den Ansprüchen 1 bis 4, dadurch gekennzeichnet, daß man das für eine stöchiometrische Zusammensetzung fehlende Aluminium als Aluminiumhydroxidgel im vorgelegten Wasser suspendiert.

6. Verfahren nach den Ansprüchen 1 bis 5, dadurch gekennzeichnet, daß das suspendierte Dihydroxialuminiumnatriumcarbonat in einem heißen Luftstrom zerstäubt und getrocknet wird.

7. Verfahren nach den Ansprüchen 1 bis 5, dadurch gekennzeichnet, daß das suspendierte Dihydroxialuminiumnatriumcarbonat abfiltriert und getrocknet wird.

8. Verfahren nach den Ansprüchen 1 bis 5, dadurch gekennzeichnet, daß das suspendierte Dihydroxialuminiumnatriumcarbonat abzentrifugiert und getrocknet wird.

9. Verfahren nach den Ansprüchen 1 bis 8, dadurch gekennzeichnet, daß das Kohlendioxid rezirkuliert wird.

## Claims

1. Process for the preparation of dihydroxyaluminium sodium carbonate by reacting aqueous sodium aluminate solutions with carbon dioxide and subsequent separation and drying of the reaction product, characterized in that at a reaction temperature between 20 and 35°C carbon dioxide is initially introduced into water up to saturation, then whilst maintaining the reaction temperature accompanied by further carbon dioxide introduction, aqueous sodium aluminate solution with a $Na_2O/Al_2O_3$ molar ratio of 1 : 1 to 1.25 : 1 is continuously added, in such a way that the pH-value during precipitation in the suspension always remains below 9.8 and preferably between 9.8 and 8.8 and finally, after pH-value setting with carbon dioxide to 7 to 7.5, the precipitation product is separated from the suspension and dried at elevated temperature.

2. Process according to claim 1, characterized in that a sodium aluminate solution with a $Na_2O/Al_2O_3$ molar ratio off 1.05 : 1 to 1.15 : 1 is used.

3. Process according to claims 1 and 2, characterized in that a sodium aluminate solution with 96 to 123 g of $Na_2O$/l and 140 to 180 g off $Al_2O_3$/l is used.

4. Process according to claims 1 to 3, characterized in that the reaction is carried out under elevated temperature.

5. Process according to claims 1 to 4, characterized in that the aluminium missing for a stoichiometric composition is suspended in water as aluminium hydroxide gel.

6. Process according to claims 1 to 5, characterized in that the suspended dihydroxyaluminium sodium carbonate is atomized and dried in a hot air flow.

7. Process according to claims 1 to 5, characterized in that the suspended dihydroxyaluminium sodium carbonate is filtered off and dried.

8. Process according to claims 1 to 5, characterized in that the suspended dihydroxyaluminium sodium carbonate is centrifuged and dried.

9. Process according to claims 1 to 8, characterized in that the carbon dioxide is recycled.

## Revendications

1. Procédé de fabrication de carbonate de dihydroxyaluminium-sodium par réaction d'une solution aqueuse d'aluminate de sodium avec du dioxyde de carbone et avec séparation consécutive et séchage du produit de réaction, caractérisé en ce que tout d'abord, à une température de réaction entre 20 et 35°C, on fait passer dans de l'eau du dioxyde de carbone jusqu'à saturation, puis, tout en maintenant la température de réaction et tout en continuant l'introduction de dioxyde de carbone, on ajoute continuellement une solution aqueuse d'aluminate de sodium ayant un rapport molaire $Na_2O/Al_2O_3$ de 1 : 1 à 1,25 : 1, de manière que la valeur du pH au cours de la précipitation dans la suspension reste constamment au-dessous de 9,8, de préférence entre 9,8 et 8,8, on sépare ensuite, après réglage de la valeur du pH avec du dioxyde de carbone à 7—7,5, le produit précipité à partir de la suspension et on le sèche à une température plus élévée.

2. Procédé selon la revendication 1, caractérisé en ce qu'on utilise une solution d'aluminate de sodium ayant un rapport molaire $Na_2O/Al_2O_3$ de 1,05 : 1 à 1,15 : 1.

3. Procédé selon les revendications 1 et 2, caractérisé en ce qu'on utilise une solution d'aluminate de sodium à

96 à 123 g de $Na_2O$/litre et à
140 à 180 g de $Al_2O_3$/litre.

4. Procédé selon les revendications 1 à 3, caractérisé en ce qu'on exécute la réaction sous pression plus élevée.

5. Procédé selon les revendications 1 à 4, caractérisé en ce que l'aluminium qui manque pour avoir une composition stoechiométrique est mis en suspension sous forme de gel d'hydroxyde d'aluminium dans l'eau introduite à l'avance.

6. Procédé selon les revendications 1 à 5, caractérisé en ce que le carbonate de dihydroxyaluminium-sodium en suspension est pulvérisé dans un courant d'air chaud et séché.

7. Procédé selon les revendications 1 à 5, caractérisé en ce que le carbonate de dihydroxyaluminium-sodium en suspension est séparé par filtration et séché.

8. Procédé selon les revendications 1 à 5, caractérisé en ce que le carbonate de dihydroxyaluminium-sodium en suspension est séparé par centrifugation et séché.

9. Procédé selon les revendications 1 à 8, caractérisé en ce que le dioxyde de carbone est recyclé.

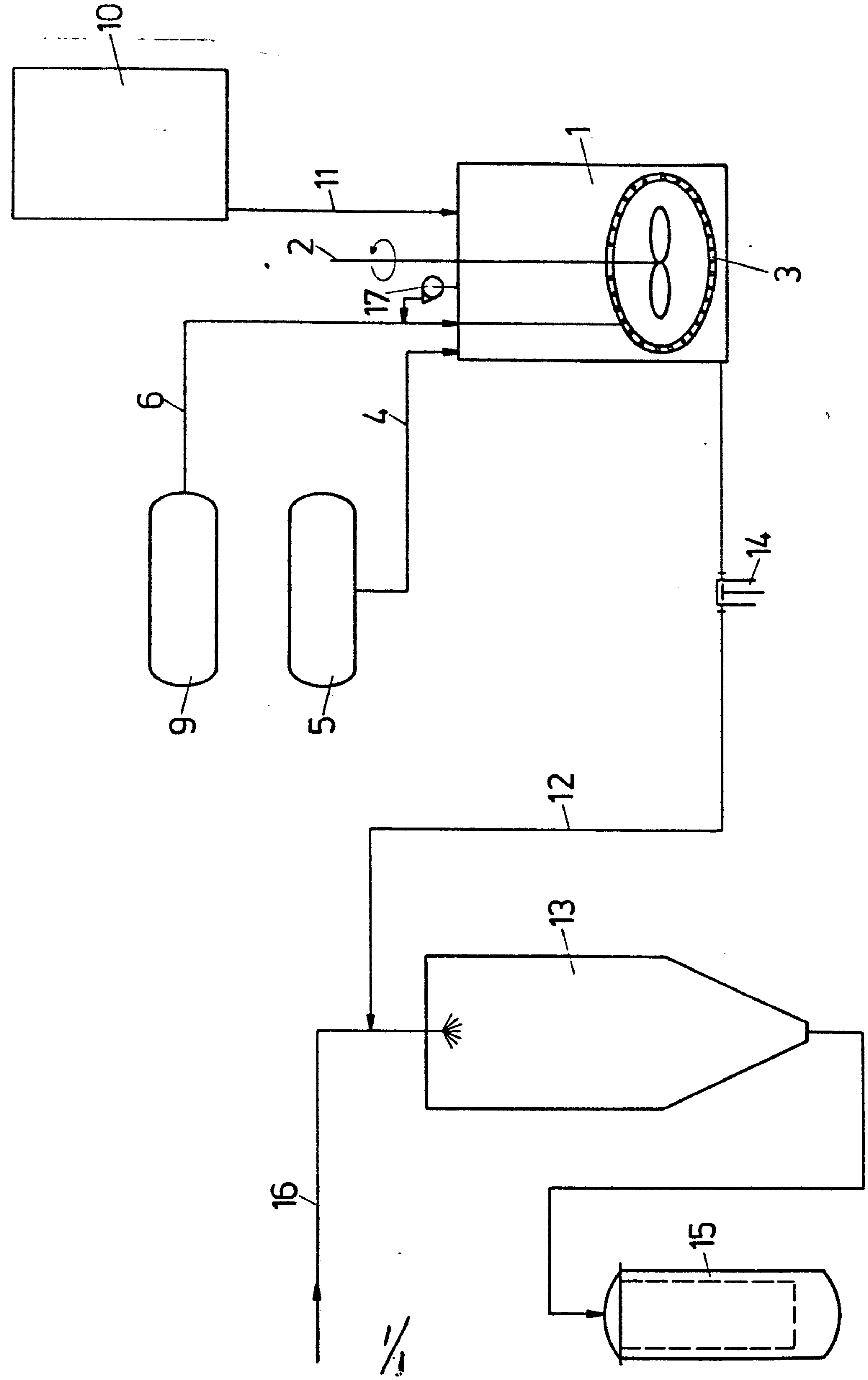
10
11
1
2
3
17
6
4
14
9
5
12
13
16
15